Europäisches Patentamt

(19) European Patent Office

Office européen des brevets

(11) Publication number: **0 118 276**
**B1**

(12) # EUROPEAN PATENT SPECIFICATION

(45) Date of publication of patent specification: **02.08.89**

(51) Int. Cl.⁴: **C 12 M 1/34**

(21) Application number: **84301280.8**

(22) Date of filing: **28.02.84**

(54) **Systems and methods for reading and recording the results of microbiological tests.**

(30) Priority: **04.03.83 US 472412**

(43) Date of publication of application:
**12.09.84 Bulletin 84/37**

(45) Publication of the grant of the patent:
**02.08.89 Bulletin 89/31**

(84) Designated Contracting States:
**AT BE CH DE FR GB IT LI LU NL SE**

(56) References cited:
**WO-A-81/03223**
**FR-A-2 350 393**
**FR-A-2 378 858**

(73) Proprietor: **AMERICAN HOME PRODUCTS
CORPORATION
685, Third Avenue
New York, New York 10017 (US)**

(72) Inventor: **Godsey, James Hal
21 East 17th Street
Huntington Station New York 11746 (US)**

(74) Representative: **Chettle, Adrian John et al
c/o John Wyeth & Brother Limited Huntercombe
Lane South
Taplow Maidenhead Berkshire, SL6 0PH (GB)**

EP 0 118 276 B1

Courier Press, Leamington Spa, England.

## Description

The present invention relates to apparatus for reading and recording the results of microbiological tests.

Microbiological tests may be carried out in test chambers or wells containing a particular reagent and/or growth medium. Test chamber holders having a plurality of test-chambers are usually provided where different combinations of reagent and/or growth medium are required.

In an existing apparatus for use in reading and manually recording such results, a transparent stage is provided on which a transparent test chamber holder or tray is placed. Light is projected from above the holder on the stage and a magnifying mirror below the stage projects a magnified image of the test chamber holder to the user who manually makes an appropriate record of the test results. The mirror is tiltable by the user to provide the best view of all test chambers.

The manual recording of a large number of test results read from a large number of closely spaced test chambers can be tedious and wasteful of the technician's time. Therefore, systems have been provided for automating at least a portion of this process. In one existing apparatus for use in semi-automatically recording the results of microbiological tests, a test tray having a plurality of test chambers arranged in a certain pattern is placed beneath a transparent keyboard. A light source projects light through the tray and the keyboard so that the user can view the tray with its test chambers through the keyboard. The keys of the keyboard correspond to the test chambers, so that the user presses the keys overlying those chambers in which certain test results have occured in order to record the results of the tests conducted in the test chambers.

The reading and interpretation of the results of microbiological test requires experience in evaluating the often subtle visual distinctions in appearance between test chambers having positive and negative test results. The intervention of a keyboard between the user and the test chamber tray makes it relatively more difficult for the user to interpret the results of the tests. As the user interprets successive test chambers, he may alter his opinion on whether a particular event in a preceding test chamber is significant or not. The prior apparatus does not allow the user to delete results already recorded. WO/03223 discloses apparatus for assisting the visual assessment of test objects having multivariate visible characteristics. In this apparatus test wells are illuminated under the control of a computer program to prevent operator error; the illumination sequence is automatic. This apparatus has the disadvantages that the user cannot choose a particular sequence of wells which may, in his experience, yield useful observations, nor may the user change his opinion on whether the result in a particular test well is significant without abandoning the sequence and starting again.

This prior art is a classic example of excessive automation of a process which in fact requires the user to make decisions based on observations of the complete sequence, and to re-assess those observations as the process continues. The apparatus of the present invention provides assistance without forcing the user to follow a pre-determined sequence which may be inappropriate. The present invention provides an improved apparatus for recording the results of microbiological tests which overcomes the aforementioned disadvantages.

According to the invention there is provided apparatus for recording the results of microbiological tests and adapted to receive a test tray having a plurality of test chambers, the apparatus including a keyboard having keys for association each with a respective test chamber and operable to actuate indicating means to indicate a respective test chamber and recording means to record the occurence of an event in a respective test chamber. Preferably the keys are touch sensitive to illuminate a light source associated with a respective test chamber and have switch means responsive to greater user presssure to actuate the recording means.

In this way the user can conveniently check that a particular key is associated with a respective test chamber before pressing the key to record an event, if desired. The keys are conveniently touch sensitive to allow the user to indicate a respective test chamber but to deactivate the light source without recording an event. The user is thus able to scan the keyboard manually to locate those keys which he wishes to press to record certain test results. Preferably the light source will remain illuminated once the event has been recorded to avoid the user attempting to duplicate a result.

The recording means is preferably operable in a first mode to provisionally record certain events and in a second mode to permanently record those events. In this way the user may delay making an unalterable record of the test until he is satisfied that the provisional results are an accurate record of the events in the test chambers.

In a preferred embodiment of the invention the apparatus includes display means to project an image of the test tray to the user. This is desirable to avoid the user looking vertically onto the test tray whilst attempting to operate a keyboard. The display means may comprise a light source above the test tray and a mirror below the test tray to project an image of the tray, which may be magnified, to the user. The keyboard may be conveniently located adjacent the mirror.

The light sources for each test chamber may be provided on an opaque apertured plate located between the test tray and the mirror. An image of each test chamber is projected through a respective aperture of the plate onto the mirror. This arrangement has the advantage that only the test chambers are projected onto the mirror; other confusing detail of the test tray being masked by the apertured plate.

Each light source may be located over an arrow shaped aperture in the plate so that, when actuated, an illuminated arrow points to the respective test chamber. A transparent template may be provided between the apertured plate and the mirror, the template carrying indicia to uniquely identify one or more test chambers. In this way the user can tell at a glance the contents of a respective test chamber. Location means are preferably provided on a casing for the aperture to hold the test tray, apertured plate and template in register.

Certain areas of the template may be occluded or opaque to mask one or more test chambers. This is desirable where a number of pre-charged test chambers remain unused in a particular test and where their presence on the mirrored image may confuse the user. The user may thus select an appropriate test chamber holder and template so that he only sees those chambers of interest when the test results are viewed.

In a further aspect of the invention a kit is provided comprising a test tray having a number of pre-charged chambers, and one or more templates for positioning in register with the test tray and having indicia to indicate the contents of certain test chambers.

The test kit combination, therefore, may be standardized for use in performing various different types of tests. In order to perform a particular test, the user only selects a template corresponding to that particular test which serves to identify only that subcombination of less than all test chambers whose results are relevant to the particular test being performed. In accordance with a preferred embodiment of the test kit combination, at least one template includes means for masking from the user's view those test chambers not included in the subcombination. By masking the chambers which are not of interest, the template eliminates a distraction to the user.

Other features of the invention are disclosed in the following description of certain preferred embodiments shown, by way of example only, with reference to the accompanying drawings, in which:

Figure 1 is a perspective view of apparatus according to the invention for use in recording the results of microbiological tests;

Figure 2 is a cross-sectional view, partially broken away, along lines 2-2 of Figure 1, and illustrating an exemplary test tray positioned for reading and recording the results of microbiological tests conducted in test chambers of the tray;

Figure 3 is a plan view of a test tray adapted for use in performing microbiological identification tests.

Figure 4 is a cross-sectional view of a test tray, partially broken away, along lines 4-4 of Figure 3;

Figure 5 is a plan view of a template for use with a test tray to indicate a subcombination of test chambers of the tray used in performing a particular microbiological test;

Figure 6 is a plan view, partially broken away, of a mask of the apparatus shown in Figure 1 having indicating means therein for indicating a test chamber selected by the user for the recording of test results; and

Figure 7 is a schematic diagram of an electrical circuit for controlling the indicating means of Figure 6 and for recording the results of the tests conducted in the chambers of the test tray.

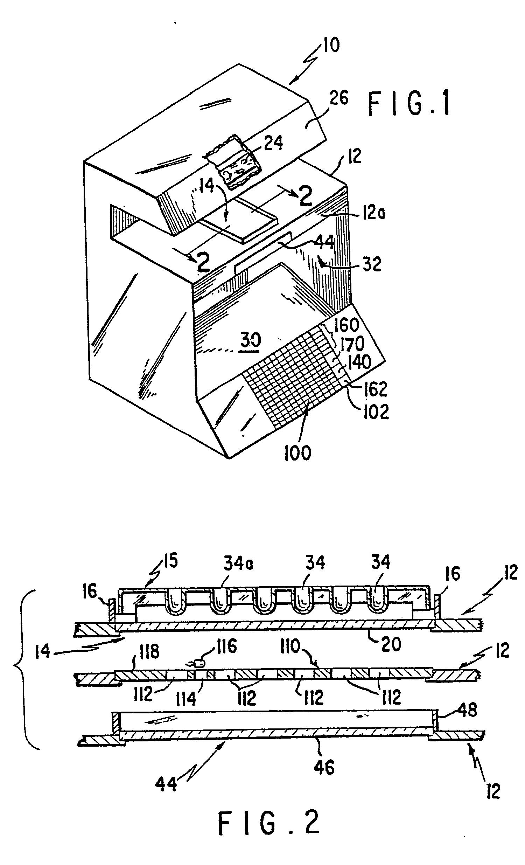

With reference first to Figures 1 and 2, a microbiological test reader and recorder 10 includes a frame 12 having a front side 12a and supporting a rectangular stage 14 having its long dimension arranged front to back with respect to frame 12 and adapted for mounting and viewing a microbiological test tray 15 to facilitate a users evaluation of tests conducted in the chambers of the tray. The stage 14 includes a holder guide 16 having four upstanding walls which serve to position the test tray 15 on the stage 14. The stage 14 also includes a transparent base 20 which both supports the tray 15 and permits an image thereof to be projected beneath the base 20, as described below. The tray 15 may alternatively be supported around the edge thereof.

Reader 10 includes a light source 24 and reflector 26 above stage 14 for projecting light downwardly through tray 15 and transparent base 20. Reader 10 also includes a magnifying mirror 30 positioned beneath base 20 to receive the image of tray 15 through base 20 and to reflect a magnified image of tray 15 to the user through an opening 32 in front side 12a. Mirror 30 is tiltably mounted on frame 12 to assume a user selectable disposition for ease of viewing. For example, mirror 30 may be tiltable between two positions: one for reading test chambers nearer the front of stage 14 and a second for reading test chambers nearer the back of the stage. Light source 24 is preferably a diffuse light source providing optimal contrast for reading susceptibility.

The test tray 15 may be, for example a microbiological test tray selected from the types disclosed in our copending European patent application 84301279.0 (EP-A- 0118275) entitled "Microbiological Test Tray". The exemplary tray 15 of Figure 2 is made of transparent plastic and includes a plurality of integrally moulded test chambers 34 arranged in a rectangular pattern. Although tray 15 is shown as having six chambers 34 across for clarity, such devices typically are constructed to have one hundred twenty test chambers arranged in a ten by twelve rectangular pattern. Where the tests to be performed are microbiological susceptibility tests (e.g. Kirby Bauer or Minimum Inhibitory Concentration (MIC) tests), each of the chambers 34 is charged with a predetermined amount of one or more antimicrobials.

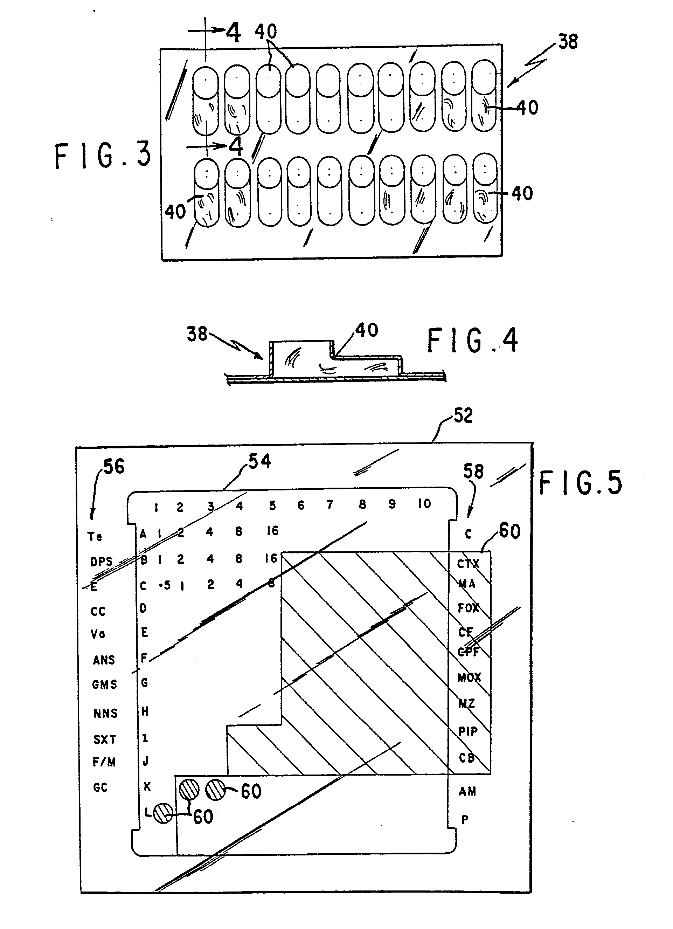

Where the tests to be performed are microbiological identification tests, an appropriate test tray, such as tray 38 of Figure 3 is utilized. Tray 38 includes twenty test chambers 40 in a ten by two configuration. Figure 4 is a cross-section of an exemplary chamber 40 and illustrates that tray 38 is constructed of two sheets of clear plastic bonded together, the upper sheet forming the side and upper walls of each chamber 40. Each chamber 40 is charged with a predetermined test substrate for use in performing a particular microbiological identification test.

The susceptibility and identification tests mentioned above are conducted by charging the test chambers with a predetermined amount of inoculum containing the organism to be tested and incubating for a prescribed period. Thereafter, the results of the tests are read and recorded with the aid of reader 10 of Figure 1. To do so, the user places the test tray on the base 20 of stage 14 and energizes the light source 24. To aid the user in identifying the particular test conducted in each test chamber 34, 40 of interest, a transparent template for use with the test tray is provided. The template is inserted by the user in a template stage 44 of the reader 10 positioned beneath the stage 14 (see Figures 1 and 2) and supported by the frame 12. The template stage 44 includes a transparent template base 46 on which the template rests and the template guide 48 having three upstanding walls which ensure registration of the template and test tray 15 as seen by the user in the mirror 30. The template may alternatively be supported around the edge thereof.

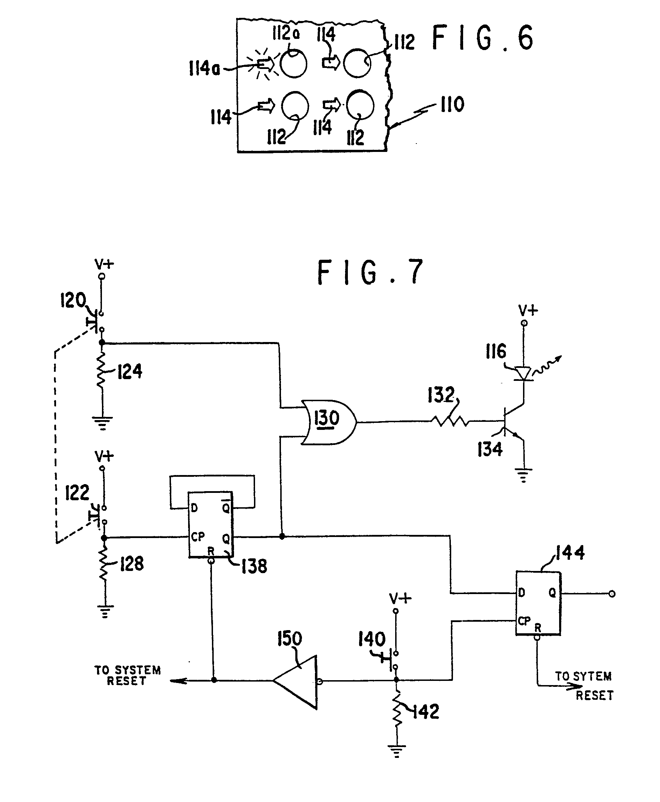

Each template is provided for use in reading the results of tests on a particular type of microorganism (such as a gram negative or gram positive organism) conducted in one or more test trays (such as trays 15 and 38). An exemplary template 52 is illustrated in Figure 5. Template 52 is constructed of transparent plastic and has a rectangular shape dimensioned to fit closely between the upstanding walls of guide 48 for registration with a test tray mounted on stage 14. The particular template 52 is adapted for use in reading the results of Minimum Inhibitory Concentration tests on gram positive bacteria conducted in a test tray having a ten by twelve array of test chambers each charged with a predetermined amount of a particular antibiotic. The construction of such a test tray is disclosed in our copending European patent application 84301279.0 (EP-A- 0118275) entitled "Microbiological Test Tray".

To aid the user in aligning the template 52 with the test tray (for example, to permit the user to read test results on a standard manual reader), an outline 54 thereof is printed on template 52. The numbers 1 through 10 arranged in a row beneath the top of outline 54 on template 52 identify the columns of test chambers in the test tray and the letters A through L on template 52 identify the rows of test chambers. Also provided on template 52 outside the outline of the test tray are two columns 56 and 58 of symbols, each symbol being arranged opposite one of the rows A to L. Each symbol (except the symbol "GC") represents a particular antibiotic contained in varying amounts in each of several adjacent test chambers of the associated one of rows A through L. The symbol "GC" represents a growth control chamber (row K, column 1) having no antibiotic therein for use as a check against the reliability of the test results; i.e., unless the organism grows in the growth control chamber, the test results in the other test chambers are not reliable.

The amount of each antibiotic in each test chamber is selected to produce a predetermined concentration of one or more antibiotics in the test chamber when it is charged with a predetermined amount of inoculum. The predetermined antibiotic-concentration(s) for each test chamber is printed on the template in registration with the position of each test-chamber in the tray when aligned with the template, so that when the aligned tray and template are viewed through the mirror 30 of reader 10, the respective concentration (in micrograms/ml) appears in the image against the background of the test chamber and its contents. For illustration, only the first five concentrations in rows A-C are provided in Figure 5 representing concentrations of the antibiotics tetracycline (symbol: Te); methicillin (symbol: $DP_s$); and erythromycin (symbol: E).

Additional antibiotics and/or varying amounts of antibiotics utilized in testing the MIC's of gram negative bacteria (but not those of gram positive bacteria) are also included in certain test chambers of the tray. These test chambers and those symbols representing antibiotics not used for testing gram positive MIC's, as well as unused test chambers (e.g. chamber L1), are masked by template 52 (as indicated by the cross-hatched areas 60 in Figure 5), so that the user in reading the test results is not distracted by irrelevant information. The antibiotic concentrations utilized in the MIC tests are selected in accordance with standards prescribed by the National Committee for Clinical Laboratory Standards (NCCLS) of the United States of America.

To enable the user to record the results of the tests as seen in the image projected by the mirror 30, the user utilizes a keyboard 100 positioned in the front side 12a of the frame 12. With the exception of the keys in a left column 102 of the keyboard 100, all of the keys thereof are arranged in a predetermined pattern corresponding to the predetermined pattern of test chambers as arranged in the test tray, so that each of the keys of keyboard 100 (except for those in column 102) represents a corresponding test chamber position of the test tray. With reference also to Figures 2 and 6, a mask 110 supported by frame 12 is positioned between stage 14 and template stage 44 of reader 10. Mask 110 has a plurality of apertures 112 each positioned in mask 110 directly beneath the position of a corresponding test chamber 34 of the test tray 15 so that only the test chambers 34 (as opposed to the remainder of tray 15) are visable in the image projected by the mirror 30. A plurality of indicator apertures 114 shaped as arrows (as seen in Figure 6) also are formed in the mask 110 (only one of the apertures 114 being shown in Figure 2). Each of apertures 114 points to and is associated with a corresponding aperture 112. A plurality of light emitting diodes (LED's) 116 are arranged on a top surface 118 of mask 110, each LED 116 being positioned over a respective aperture 114 so that when each LED 116 is illuminated, its image may be seen through the corresponding arrow shaped aperture 114 in the image projected by the mirror 30 to indicate its corresponding aperture 112 and hence chamber 34. For example the illumination of the LED 116 corresponding to arrow shaped aperture 114a in Figure 6 indicates the corresponding aperture 112a.

The illumination of each of the LED's 116 is controlled by the user through the activation of a respective key in keyboard 100 whose position corresponds to the position of the aperture 112 to which the particular indicator aperture 114 associated with the LED 116 points. Accordingly, the user's activation of a given key of keyboard 100 (with the exception of the keys in column 102 thereof) controls the illumination of an LED 116 to indicate the test chamber of the test tray corresponding to the key activated by the user. Figure 7 is a schematic diagram of a circuit which controls the operation of a particular LED 116 in response to the activation of a particular key of the keyboard 100. The circuit of Figure 7 includes two momentary contact switches 120 and 122 which are ganged for mutual operation by the key of keyboard 100 corresponding to the particular LED 116 under the control of the circuit of Figure 7. Switch 120 is responsive either to a very light touch of the associated key or a relatively small pressure applied thereto to close, whereas switch 122 is responsive to a relatively greater pressure exceeding a predetermined threshold level to close. Accordingly, the application of pressure less than this threshold value to the associated key will close only switch 120 while switch 122 remains open; whereas the application of pressure to the associated key in excess of the threshold level will cause both of switches 120 and 122 to close. Switch 120 may be, for example, a touch sensitive membrane switch.

A first terminal of each switch 120 and 122 is connected to a source of positive voltage V+ and a second terminal of each switch 120 and 122 is coupled to ground through a respective resistor 124, 128. Therefore, the second terminal of each of switches 120 and 122 is effectively at ground potential when the respective switch is open and at the voltage level V+ when the respective switch is closed.

Switch 120 serves to energize the associated LED 116 so long as switch 120 is closed. This provides the user with a convenient means for indicating the test chamber associated with the particular key he is touching, since his touch of the key will close switch 120 to illuminate the associated LED 116 which in turn provides a visual indication of the corresponding test chamber appearing in the image displayed in the mirror 30. The energization of LED 116 in response to the activation of switch 120 is achieved in the circuit of Figure 7 as follows: a second terminal of switch 120 is coupled to a first input terminal of an OR gate 130 and the output of OR gate 130 is coupled through a resistor 132 to the base of an NPN transistor 134. The emitter of transistor 134 is coupled to ground while its collector is coupled to the cathode of LED 116. The anode of LED 116 is connected to the source of positive voltage V+. Therefore, when switch 120 is closed in response to the user's touch of the corresponding key, the first input terminal of OR gate 130 will be raised to V+, thus to force the output of OR gate 130 high, turning on transistor 134 and energizing LED 116.

Switch 122 serves as a means for recording the occurence of certain test results in the test chamber associated therewith. For example, if the user desires to record the occurence of a positive test result in the associated test chamber, he would apply sufficient pressure to the key to cause switch 122 to close. The second terminal of switch 122 is coupled to the clock input terminal of a D-type flip flop 138. Flip flop 138 has its $\bar{Q}$ terminal connected to its D terminal so that the state of the Q terminal of the flip flop toggles with each application of a pulse to the clock input thereof through the activation of switch 122. It will be appreciated that appropriate debouncing circuitry may be provided in connection with switch 122 to avoid multiple toggling of flip flop 138 in response to a single depression of the associated key by the user.

The present state of flip flop 138 is indicated conveniently by the associated LED 116, which is achieved by coupling the Q terminal of the flip flop 138 to a second input terminal of OR gate 130, so that when the Q terminal of flip flop 138 is high, LED 116 will be illuminated, while the LED 116 will not be illuminated if the Q terminal is low (provided switch 120 is not activated). It will be appreciated that the user may also, if desired, change a previously recorded test result simply by reactivating switch 122 which will in turn cause flip flop 138 to toggle again, thus to record the opposite test result.

The above described circuitry of Figure 7 is duplicated for each key of the keyboard 100 associated with a particular test chamber position and each LED 116. When the user is satisfied that he has properly recorded the test results for all relevant test chambers, he then activates a data entry switch 140 of keyboard 100 which causes the system to enter a second mode of operation wherein the test results are stored inalterably for further data processing by the reader 10 and by other associated data processing apparatus. As seen in Figure 7, switch 140 is a momentary contact switch having a first terminal connected to V+ and a second terminal coupled through a resistor 142 to ground. The second terminal of switch 140 is coupled to the clock input of a second D type flip flop 144 whose D input is connected to the Q terminal of flip flop 138. Accordingly, the activation of switch 140 will clock the state of the Q terminal of flip flop 138 into flip flop 144 so that its Q terminal assumes the present state of the Q terminal of the flip flop 138.

The second terminal of switch 140 is coupled to the input of an inverter 150 whose output terminal is coupled to a reset terminal of flip flop 138 (active low). Accordingly, the activation of switch 140 will reset flip flop 138 for recording other test results after a period of time representing the propagation delay of inverter 150 during which the state of flip flop 138 is clocked into flip flop 144. The second terminal of switch 140 is also coupled to the associated circuitry of the remaining keys of keyboard 100 (with appropriate buffering) to effect data storage in all of the corresponding flip flops 144 as well as a reset of all flip flops 138 simultaneously. In an alternative embodiment, data entry switch 140 controls only a subcombination of less than all circuits. A second data entry switch 170 is connected to a second respective subcombination of circuits such as that of Figure 7 in the same manner as switch 140 to perform the identical functions but only for the associated circuits of its subcombination. For example, switch 140 may serve to inalterably record only susceptibility test results which would be performed in test chambers associated with the circuits controlled by switch 140, whereas switch 170 would serve to inalterably record only identification test results which have been read from test chambers associated with the circuits controlled by switch 170.

The switch of keyboard 100 associated with the growth control test chamber serves as a means for the user to initiate the reading and recording of a new series of tests. For example, the user places a new test tray in the stage 14 and checks the growth control well to ascertain that the organism has indeed grown therein. If so, he activates the growth control switch which effects a system reset which is received by each of the flip flops 138 and 140 on its reset terminal (active low) as a low going pulse which thus effects a reset of all flip flops 138 and 144 thus to reinitialize the system.

In the case of microbiological identification tests, it is frequently necessary to perform auxiliary tests in devices not adapted to be read optically by the reader 10. It is, nevertheless, desirable to record the results of such tests simultaneously with the remaining tests performed in the test tray which can be read in reader 10. For this purpose, auxiliary data entry switches 160 and 162 in column 102 of keyboard 100 are provided. These switches 160 and 162 may, for example, also be momentary contact switches each serving to toggle an associated D type flip flop when activated and to illuminate an associated LED positioned in the switch key to inform the user of the present state of the associated flip flop. Each flip flop associated with the switches 160 and 162 is likewise read and reset when the controlling data entry switch 140 or 170 is activated by the user.

A microcomputer system may be provided to read and store the states ofthe flip flop 144 subsequent to the storage of test data therein for the purpose of data reduction and communication to other data processing systems for the development of susceptibility and identification profiles in a conventional manner. It will also be appreciated that the functions performed by the individual circuits of Figure 7 may likewise be implemented in a microprocessor controlled system.

The terms and expressions which have been employed are used as terms of description and not of limitation, and there is no intention in the use of such terms and expressions of excluding an equivalents of the features shown and described, or any portion thereof, it being recognised that various modifications are possible within the scope of the invention claimed.

**Claims**

1. Apparatus for recording the results of microbiological tests and adapted to receive a test tray (15, 38) having a plurality of test chambers (34, 40), the apparatus including a keyboard (100) having keys for association each with a respective test chamber and operable to activate recording means (122, 128, 138, 140, 150, 170) to record the occurence of an event in a respective test chamber characterised thereby that the apparatus (10) further includes indicating means (114, 116) responsive to actuation of a key to indicate a respective test chamber (34, 40) only whilst said key is actuated.

2. Apparatus according to Claim 1, characterised thereby that said keys are resposive to a first pressure to activate said indicating means (114, 116) and to a second, higher pressure to activate said recording means (122, 128, 138, 140, 150, 170).

3. Apparatus according to Claim 1 or Claim 2, characterised thereby that said recording means (122, 128, 138, 140, 150, 170) is operable in a first mode to provisionally record an event and in a second mode to permanently record the event.

4. Apparatus according to Claim 3, characterised thereby that operation of the recording means (122, 128, 138, 140, 150, 170) in said second mode de-activates said indicating means.

5. Apparatus according to any preceding claim, characterised thereby that said indicating means comprise a plurality of light sources (116), one each for association with a respective test chamber (34, 40), said keys being touch sensitive to illuminate an associated light source.

6. Apparatus according to any preceding claim, characterised thereby that the apparatus further includes display means (24, 26, 30) for displaying an image of said tray (15, 38) and indicating means (114, 116).

7. Apparatus according to Claim 6, characterised thereby that said display means (24, 26, 30) further includes means for superimposing on said image indicia uniquely identifying one or more test chambers.

8. Apparatus according to Claim 7, characterised thereby that said indicia are provided on a transparent template (52), location means (48) being provided to hold the template (52) in register with said test tray (15, 38).

9. Apparatus according to Claim 8, characterised thereby that said indicating means (114, 116) are provided on an apertured plate (118), the test tray (15, 38), apertured plate (118) and template (52) being arranged in register one above the other; a light source (24) and a reflector (26) being provided on one side of the test tray and a mirror (30) on the other side thereof such that an image of each test chamber (34, 40) is displayed on said mirror (30) through a respective aperture (112) of said plate (118).

10. Apparatus according to Claim 8 or Claim 9, characterised thereby that occluded areas (60) are provided on said template to mask one or more test chambers.

**Patentansprüche**

1. Vorrichtung zum Registrieren der Ergebnisse von mikrobiologischen Untersuchungen und eingerichtet zur Aufnahme einer Testschale (15, 38) mit einer Mehrzahl von Testkammern (34, 40), wobei die Vorrichtung eine Tastatur (100) mit Tasten enthält, die je einer zugehörigen Testkammer zugeordnet sind und betätigbar sind, um eine Registriereinrichtung (122, 128, 138, 140, 150, 170) zum Registrieren des Auftretens eines Ereignisses in einer entsprechenden Testkammer zu aktivieren, dadurch gekennzeichnet, daß die Vorrichtung (10) weiters auf die Betätigung einer Taste ansprechende Anzeigemittel (114, 116) zur Anzeige einer entsprechenden Testkammer (34, 40) nur, während die genannte Taste betätigt ist, aufweist.

2. Vorrichtung nach Anspruch 1, dadurch gekennzeichnet, daß die Tasten auf einen ersten Druck, um die Anzeigemitel (114, 116) zu aktivieren, und auf einen zweiten, höheren Druck ansprechen, um die Registriereinrichtung (122, 128, 138, 140, 150, 170) zu aktivieren

3. Vorrichtung nach Anspruch 1 oder 2, dadurch gekennzeichnet, daß die Registriereinrichtung (122, 128, 138, 140, 150, 170) in einer ersten Betriebsart, um ein Ereignis vorläufig zu registrieren, und in einer zweiten Betriebsart betätigbar ist, um das Ergebnis bleibend zu registrieren.

4. Vorrichtung nach Anspruch 3, dadurch gekennzeichnet, daß die Betätigung der Registriereinrichtung (122, 128, 138, 140, 150, 170) in der zweiten Betriebsart die Anzeigemittel außer Funktion setzt.

5. Vorrichtung nach einem der vorhergehenden Ansprüche, dadurch gekennzeichnet, daß die Anzeigemittel eine Mehrzahl von Lichtquellen (116) umfassen, die je einer entsprechenden Testkammer (34, 40) zuzuordnen sind, wobei die Tasten berührungsempfindlich sind, um eine zugehörige Lichtquell leuchten zu lassen.

6. Vorrichtung nach einem der vorstehenden Ansprüche dadurch gekennzeichnet, daß die Vorrichtung weiters eine Wiedergabeeinrichtung (24, 26, 30) zur Wiedergabe eines Bildes der Schale (15, 38) und Anzeigemittel (114, 116) enthält.

7. Vorrichtung nach Anpruch 6, dadurch gekennzeichnet, daß die Wiedergabeeinrichtung (24, 26, 30) weiters Mittel zum Überlagern von Kennzeichen, die eindeutig eine oder mehrere Testkammern identifizieren, auf dem Bild enthält.

8. Vorrichtung nach Anspruch 7, dadurch gekennzeichnet, daß die Kennzeichen auf einer transparenten Matrize (52) vorgesehen sind, wobei Lagemittel (48) vorgesehen sind, um die Matrize (52) in Ausrichtung zur Testschale (15, 38) zu halten.

9. Vorrichtung nach Anspruch 8, dadurch gekennzeichnet, daß die Anzeigemittel (114, 116) auf einer mit Öffnungen versehenen Platte (118) vorgesehen sind, wobei die Testschale (15, 38), die mit Öffnungen versehene Platte (118) und die Matrize (52) in Ausrichtung übereinander angeordnet sind; eine Lichquelle (24) und ein Reflektor (26) n einer Seite der Testschale und ein Spiegel (30) an der anderen Seite hievon derart vorgesehen sind, daß ein Bild jeden Testkammer (34, 40) auf dem Spiegel (30) durch eine entsprechende Öffnung (112) der Platte (118) hindurch wiedergegeben wird.

10. Vorrichtung nach Anspruch 8 oder 9, dadurch gekennzeichnet, daß abgedeckte Bereiche (60) auf der Matrize vorgesehen sind, um eine oder mehrere Testkammern zu maskieren.

**Revendications**

1. Appareil pour enregistrer les résultats de tests microbiologiques, propre à recevoir une plaque pour tests (15, 38) comportant un grand nombre de chambres de tests (34, 40), cet appareil comprenant un clavier (100) comportant des touches destinées à être, associées chacune a une chambre de test respective et pouvant être actionnées pour activer des moyens d'enregistrement (122, 128, 138, 140, 150, 170) pour enregistrer l'apparition d'un événement dans une chambre de test respective, caractérisé en ce qu'il comprend, en outre, des moyens indicateurs (114, 116) réagissant à l'actionnement d'une touche pour désigner une chambre de test respective (34, 40) uniquement pendant que la touche est actionnée.

2. Appareil suivant la revendication 1, caractérisé en ce que les touches réagissent à une première pression pour activer les moyens indicateurs (114, 116) et à une seconde pression plus élevée pour activer les moyens d'enregistrement (122, 128, 138, 140, 150, 170).

3. Appareil suivant la revendication 1 ou 2, caractérisé en ce que les moyens d-enregistrement (122, 128, 138, 140, 150, 170) peuvent fonctionner dans un premier mode pour enregistrer provisoirement un événement et, dans un second mode, pour enregistrer l'événement de manière permanente.

4. Appareil suivant la revendication 3, caractérisé en ce que le fonctionnement des moyens d'enregistrement (122, 128, 138, 140, 150, 170) dans le second mode désactive les moyens indicateurs.

5. Appareil suivant l'une quelconque des revendications précédentes, caractérisé en ce que les moyens indicateurs comprennent plusieurs sources lumineuses (116), chacune d'elles étant associée à une chambre de test respective (34, 40), les touches étant des touches à effleurement destinées à allumer une source lumineuse associée.

6. Appareil suivant l'une quelconque des revendications précédentes, caractérisé en ce qu'il comprend, en outre, un moyen de visualisation (24, 26, 30) pour visualiser une image de la plaque (15, 38) et des moyens indicateurs (114, 116).

7. Appareil suivant la revendication 6, caractérisé en ce que le moyen de visualisation (24, 26, 30) comprend, en outre, un moyen pour superposer a l'image des signes identifiant de manière unique une ou plusieurs chambres de tests.

8. Appareil suivant la revendication 7, caractérisé en ce que les signes sont prévus sur un gabarit transparent (52), des moyens de positionnement (48) étant prévus pour maintenir le gabarit (52) en coïncidence avec la plaque pour tests (15, 38).

9. Appareil suivant la revendication 8, caractérisé en ce que les moyens indicateurs (114, 116) sont prévus sur une plaque à ouvertures (118), la plaque pour tests (15, 38), la plaque à ouvertures (118) et le gabarit (52) étant disposés en coïncidence l'un au-dessus de l'autre, une source lumineuse (24) et un réflecteur (26) étant prévus d'un côté de la plaque pour tests et un miroir (30) étant prévu de l'autre côté de sorte qu'une image de chaque chambre de test (34, 40) est projetée sur le miroir (30) à travers une ouverture respective (112) de la plaque (118).

10. Appareil suivant la revendication 8 ou 9, caractérisé en ce que des zones occluses (60) sont prévues sur le gabarit pour masquer une ou plusieurs chambres de tests.

EP 0 118 276 B1

FIG.1

FIG.2

FIG.3

4  40                    38
                              40
4
                              40
40                            40

38          40    FIG.4

52

56          54          58    FIG.5
                                    60

        1  2  3  4  5  6  7  8  9  10

Te   A  1  2  4  8  16              C
DPS  B  1  2  4  8  16              CTX
E    C  ·5  1  2  4  8              MA
CC   D                             FOX
Va   E                             CF
ANS  F                             GPF
GMS  G                             MOX
NNS  H                             MZ
SXT  I                             PIP
F/M  J                             CB
GC   K                             AM
     L                             P

              60
60

EP 0 118 276 B1

FIG.6

FIG.7